# EUROPÄISCHE PATENTSCHRIFT

(11) **EP 0 502 138 B1**
(45) Veröffentlichungstag und Bekanntmachung des Hinweises auf die Patenterteilung: **20.09.1995**
(21) Anmeldenummer: 91914991.4
(22) Anmeldetag: 17.08.1991
(51) Int. Cl.: A61K 7/13, C07C 211/52

(54) **MITTEL ZUR OXIDATIVEN FÄRBUNG VON HAAREN UND NEUE 5-HALOGEN-2,4-DIAMINOALKYLBENZOLE**
OXIDATIVE HAIR-DYEING AGENT AND NEW 5-HALO-2,4-DIAMINOALKYLBENZENES
AGENT DE COLORATION DES CHEVEUX PAR OXYDATION ET NOUVEAUX 5-HALOGEN-2,4-DIAMINOALKYLBENZENES

(30) Priorität: 10.09.1990 DE 4028661
(43) Veröffentlichungstag der Anmeldung: 09.09.1992
(73) Patentinhaber: Wella Aktiengesellschaft, 64295 Darmstadt (DE)
(72) Erfinder: CLAUSEN, Thomas, D-6146 Alsbach (DE); WEINGES, Alexa, D-6900 Heidelberg (DE); BALZER, Wolfgang, R., D-6146 Alsbach (DE)
(86) Internationale Anmeldenummer: EP9101565
(87) Internationale Veröffentlichungsnummer: WO9204005

(56) Entgegenhaltungen:
- EP-A- 0 252 351
- FR-A- 2 542 193
- GB-A- 2 054 666
- US-A- 4 031 160

## Beschreibung

Gegenstand der Erfindung ist ein Mittel zur oxidativen Färbung von Haaren auf der Basis von 5-Halogen-2,4-diaminoalkylbenzolen als Kupplersubstanzen sowie neue 5-Halogen-2,4-diaminoalkylbenzole.

In der Haarfärbepraxis haben Oxidationsfarbstoffe eine wesentliche Bedeutung erlangt. Hierbei werden die Farbstoffe durch oxidative Kupplung von Entwicklersubstanzen und Kupplersubstanzen im Haarschaft erzeugt. Dies führt zu sehr intensiven Haarfärbungen mit sehr guter Farbechtheit. Außerdem können durch die Kombination geeigneter Entwickler- und Kupplersubstanzen unterschiedliche Farbnuancen erzeugt werden.

Als Entwicklersubstanzen werden bevorzugt 2,5-Diaminotoluol, 1,4-Diaminobenzol, 2-(2'-Hydroxyethyl)-1,4-diaminobenzol, 4-Aminophenol, 4-Amino-2-aminomethylphenol und 4-Amino-3-methylphenol verwendet.

Die bevorzugt verwendeten Kupplersubstanzen sind dabei m-Phenylendiamin und dessen Derivate, wie zum Beispiel 2,4-Diamino-phenoxyethanol, 2,4-Diaminobenzylalkohol, 2-Amino-4-(2'-hydroxyethyl)aminoanisol oder Pyridinderivate wie 3,5-Diamino-2,6-dimethoxypyridin als Blaukuppler, 1-Naphthol, m-Aminophenol und dessen Derivate, wie 2-Amino-4-chlor-6-methylphenol, 3-Amino-2-methylphenol, 4-Amino-2-hydroxyphenoxyethanol und 3-Amino-5-hydroxy-2,6-dimethoxypyridin, als Rotkuppler sowie Resorcin, 4-Chlorresorcin, 4,6-Dichlorresorcin, 2-Methylresorcin, 4-Hydroxy-1,2-methylendioxybenzol, 4-(2'-Hydroxyethyl)amino-1,2-methylendioxybenzol und 4-Hydroxyindol als Kuppler für den Braun-Blond-Bereich.

An Oxidationsfarbstoffe, die zur Färbung menschlicher Haare Verwendung finden, sind zahlreiche besondere Anforderungen gestellt. So müssen sie in toxikologischer und dermatologischer Hinsicht unbedenklich sein und Färbungen in der gewünschten Intensität ermöglichen. Ferner wird für die erzielten Haarfärbungen eine gute Licht-, Dauerwell-, Säure- und Reibechtheit gefordert. Auf jeden Fall aber müssen solche Haarfärbungen ohne Einwirkung von Licht, Reibung und chemischen Mitteln über einen Zeitraum von mindestens 4 bis 6 Wochen stabil bleiben. Außerdem ist es erforderlich, daß durch Kombination geeigneter Entwicklerund Kupplerkomponenten eine breite Palette verschiedener Farbnuancen erzeugt werden kann.

Die zur Zeit in Haarfärbemitteln zur Erzeugung insbesondere von roten und klaren blauen Farbtönen verwendeten Kupplersubstanzen können die vorstehend genannten Anforderungen jedoch nicht zufriedenstellend erfüllen.

Das von F. Reverdin und P. Crépieux in Chemische Berichte 33, 2505 (1900) beschriebene 5-Chlor-2,4-diaminotoluol ergibt zwar mit Entwicklern vom Typ des p-Phenylendiamins reine, blaue Ausfärbungen; mit Entwicklern wie p-Aminophenol oder dessen Derivaten werden aber nur äußerst schwache, rötliche Ausfärbungen erhalten.

Das aus der DE-OS 34 30 513 bekannte 2,4-Diamino-5-tetrafluorethoxytoluol zeigt zwar im Amestest keine mutagene Wirkung, jedoch sind Farbtiefe und Lichtechtheit, der mit dieser Kupplersubstanz erzielten Ausfärbungen nicht zufriedenstellend.

Die in der DE-OS 36 22 784 beschriebenen Kupplersubstanzen 2,4-Diamino-5-ethoxytoluol beziehungsweise 2,4-Diamino-5-(2'-hydroxyethyl)oxytoluol besitzen zwar gute toxikologische und anwendungstechnische Eigenschaften, die Synthese dieser Verbindungen, welche vom 3-Hydroxy-4-nitro-toluol ausgeht, ist jedoch aufwendig, da fünf Reaktionsschritte durchgeführt werden müssen, und ist daher hinsichtlich der Synthesededauer (Lohnkosten) sowie der Energiekosten nicht zufriedenstellend.

Demgemäß besteht die Erfindungsaufgabe darin, ein Haarfärbemittel zur oxidativen Färbung von Haaren auf der Basis von in der Haarfärbung üblichen Entwicklersubstanzen mit einem Gehalt an neuen Kupplersubstanzen zur Verfügung zu stellen, bei dem die zuvor genannten gestellten Anforderungen an die anwendungstechnischen Eigenschaften und das Herstellungsverfahren der neuen Kupplersubstanz erfüllt werden.

Hierzu wurde nun gefunden, daß ein Mittel zur oxidativen Färbung von Haaren auf der Basis einer Entwicklersubstanz-Kupplersubstanz-Kombination sowie gegebenenfalls anderer Farbkomponenten und in Haarfärbemitteln üblichen Zusätzen, welches mindestens eine Kupplersubstanz der allgemeinen Formel 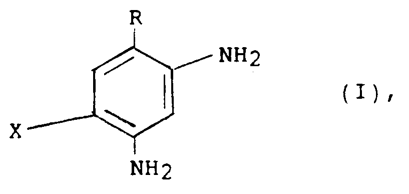
wobei R einen geradkettigen oder verzweigten C₁- bis C₄-Alkylrest und X Fluor oder Brom darstellt, oder deren physiologisch verträgliches wasserlösliches Salz enthält, die gestellte Aufgabe in hervorragendem Maß löst.

Die Kupplersubstanzen der allgemeinen Formel (I) sind durch einfache chemische Reaktionen aus technisch zugänglichen Ausgangsverbindungen erhältlich und ergeben mit Entwicklersubstanzen wie p-Phenylendiamin klare blaue Ausfärbungen ohne Rotstich. Mit Entwicklersubstanzen vom Typ des p-Aminophenol, lassen sich farbsatte, rote Färbungen erzielen, deren Farbintensität sogar größer ist als bei Ausfärbungen mit dem unsubstituierten 2,4-Diaminotoluol.

Die Kupplersubstanzen der allgemeinen Formel (I) sind gut in Wasser löslich und weisen, insbesondere als Bestandteil der hier beschriebenen Haarfärbemittel, eine ausgezeichnete Lagerstabilität auf.

In dem hier beschriebenen Haarfärbemittel sollen die erfindungsgemäßen Kupplersubstanzen der Formel (I), in einer für die Haarfärbung ausreichenden Menge, vorzugsweise in einer Menge von 0,01 bis 5 Gewichtsprozent, besonders bevorzugt 0,1 bis 3 Gewichtsprozent, enthalten sein.

Von den Kupplersubstanzen der allgemeinen Formel (I) sind in dem Haarfärbemittel bevorzugt 5-Brom-2,4-diaminotoluol und 5-Fluor-2,4-diaminotoluol enthalten.

Außerdem können in dem Haarfärbemittel zusätzlich 0,01 bis 5 Gewichtsprozent, bevorzugt 0,1 bis 3 Gewichtsprozent, mindestens einer weiteren Kupplersubstanz, beispielsweise Resorcin, 4-Chlorresorcin, 4,6-Dichlorresorcin, 2-Methylresorcin, 2-Amino-4-(2'hydroxyethylamino)anisol, 2-Amino-4-ethylamino-anisol, 2,4-Diaminobenzylalkohol, m-Phenylendiamin, 5-Amino-2-methylphenol, 2,4-Diamino-phenoxyethanol, 1-Naphthol, m-Aminophenol, 3-Amino-4-chlor-6-methylphenol, 3-Amino-2-methylphenol, 4-Amino-2-hydroxyphenoxyethanol, 4-Hydroxy-1,2-methylendioxybenzol, 4-(2'-Hydroxyethylamino)-1,2-methylendioxybenzol, 2,4-Diamino-5-ethoxytoluol, 4-Hydroxyindol, 3-Amino-5-hydroxy-2,6-dimethoxypyridin und 3,5-Diamino-2,6-dimethoxypyridin, enthalten sein.

Von den Entwicklersubstanzen kommen als Bestandteil der erfindungsgemäßen Haarfärbemittel vor allem 1,4-Diaminobenzol, 2,5-Diaminotoluol, 2,5-Diaminobenzylalkohol, 2-(2'-Hydroxyethyl)-1,4-diaminobenzol, 4-Aminophenol, 4-Amino-2-aminomethyl-phenol, 4-Amino-3-methylphenol, 4-Amino-2methoxymethylphenol, 4-Amino-2-ethoxymethylphenol sowie Tetraaminopyridin oder deren physiologisch verträglichen Salze in Betracht. Die in dem erfindungsgemäßen Mittel enthaltene Menge der Entwicklersubstanzen soll vorzugsweise 0,01 bis 5 Gewichtsprozent, besonders bevorzugt jedoch 0,1 bis 3,0 Gewichtsprozent, betragen.

Die üblichen Kuppler- und Entwicklersubstanzen können in dem erfindungsgemäßen Haarfärbemittel jeweils einzeln oder im Gemisch miteinander enthalten sein. Die Gesamtmenge der in dem hier beschriebenen Haarfärbemittel enthaltenen Entwicklersubstanz-Kupplersubstanz-Kombination soll etwa 0,1 bis 5,0 Gewichtsprozent, vorzugsweise 0,5 bis 4,0 Gewichtsprozent, betragen.

Die Entwicklersubstanzen werden im allgemeinen in etwa äquimolaren Mengen, bezogen auf die Kupplersubstanzen, eingesetzt. Es ist jedoch nicht nachteilig, wenn die Entwicklersubstanzen diesbezüglich in einem gewissen Überschuß oder Unterschuß vorhanden ist.

Weiterhin kann das Haarfärbemittel dieser Anmeldung zusätzlich auch andere Farbkomponenten, beispielsweise 6-Amino-methylphenol und 2-Amino-5-methylphenol sowie ferner übliche direktziehende Farbstoffe, zum Beispiel Triphenylmethanfarbstoffe wie Basic Violet 14 (C. I. 42 510) und Basic Violet 2 (C. I. 42 520), aromatische Nitrofarbstoffe wie 2-Nitro-1,4-diaminobenzol, 2-Amino-4-nitrophenol, 2-Amino-5-nitrophenol, 2-Amino-4,6-dinitrophenol, 2-Amino-5-(2'-hydroxyethylamino)nitrobenzol und 4-(2'-Hydroxyethylamino)-3-nitrotoluol, 1-(2'-Ureidoethyl)amino-4-nitrobenzol und Azofarbstoffe wie Acid Brown 4 (C. I. 14 805) sowie Dispersionsfarbstoffe, wie beispielsweise 1,4-Diaminoanthrachinon und 1,4,5,8-Tetraaminoanthrachinon, enthalten.

Weitere geeignete direkt auf das Haar aufziehende Farbstoffe sind beispielsweise im Buch von J. C. Johnson "Hair Dyes", Noyes Data Corp., Park Ridge, USA (1973), Seiten 3 - 91 und 113 - 139 (ISBN: 0-8155-0477-2) beschrieben.

Selbstverständlich können die Kuppler- und Entwicklersubstanzen sowie die anderen Farbkomponenten, sofern es Basen sind, auch in Form der physiologisch verträglichen Säureadditionssalze, wie beispielsweise als Hydrochlorid beziehungsweise Sulfat oder - sofern sie aromatische OH-Gruppen besitzen - in Form der Salze mit Basen, zum Beispiel als Alkaliphenolate, eingesetzt werden.

Das Haarfärbemittel kann ferner direktziehende Farbstoffe und mit sich selbst kuppelnde Farbstoffvorstufen in einer Menge von 0,1 bis 4,0 Gewichtsprozent enthalten.

Darüber hinaus können in dem Haarfärbemittel noch übliche kosmetische Zusätze, beispielsweise Antioxidantien wie Ascorbinsäure, Thioglykolsäure oder Natriumsulfit, Parfümöle, Komplexbildner, Netzmittel, Emulgatoren, Verdicker, Pflegestoffe und andere vorhanden sein.

Die Zubereitungsform kann beispielsweise eine Lösung, insbesondere eine wäßrige-alkholische Lösung, sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, Propanol und Isopropanol oder Glykole wie Glycerin und 1,2-Propylenglykol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Je nach Zusammensetzung kann das erfindungsgemäße Haarfärbemittel schwach sauer, neutral oder alkalisch reagieren. Insbesondere weist es einen pH-Wert im alkalischen Bereich zwischen 8,0 bis 11,5 auf, wobei die Einstellung vorzugsweise mit Ammoniak erfolgt. Es können aber auch organische Amine, zum Beispiel Monoethanolamin und Triethanolamin, oder auch anorganische Basen wie Natriumhydroxid und Kaliumhydroxid, Verwendung finden.

Für die Anwendung zur oxidativen Färbung von Haaren vermischt man das vorstehend beschriebenen Haarfärbemittel unmittelbar vor dem Gebrauch mit einem Oxidationsmittel und trägt eine für die Haarfärbebehandlung ausreichende Menge, je nach Haarfülle, im allgemeinen etwa 60 bis 200 g, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin oder Natriumborat in Form der 3 bis 12 %igen, vorzugsweise 6 %igen wäßrigen Lösungen, in Betracht.

Wird eine 6 %ige Wasserstoffperoxidlösung als Oxidationsmittel verwendet, so beträgt das Gewichtsverhältnis zwischen Haarfärbemittel und Oxidationsmittel 5 : 1 bis 1 : 2, vorzugsweise jedoch 1 : 1. Größere Mengen an Oxidationsmittel werden vor allem bei höheren Farbstoffkonzentrationen im Haarfärbemittel oder wenn gleichzeitig eine stärkere Bleichung des Haares beabsichtigt ist, verwendet.

Man läßt das Gemisch bei 15 bis 50 °C etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Das erfindungsgemäße Haarfärbemittel führt zu Haarfärbungen mit ausgezeichneten Echtheitseigenschaften, insbesondere was die Licht-, Wasch- und Reibechtheit anbetrifft, und läßt sich mit Reduktionsmitteln wieder abziehen. Hinsichtlich der färberischen Möglichkeiten bietet das erfindungsgemäße Haarfärbemittel je nach Art und Zusammensetzung der Farkomponenten eine breite Palette verschiedener Farbnuancen. Bemerkenswert ist die große Farbintensität der erzielbaren roten und die Farbreinheit der erzielbaren blauen Färbungen. Schließlich ist mit Hilfe des beschriebenen Haarfärbemittels auch eine Anfärbung von ergrauten und chemisch nicht vorgeschädigtem Haar problemlos und mit sehr guter Deckkraft möglich. Die dabei erhaltenen Färbungen sind, unabhängig von der unterschiedlichen Struktur des Haares, gleichmäßig und sehr gut reproduzierbar.

Die 5-Halogen-2,4-diamino-alkylbenzole der allgemeinen Formel (I) lassen sich einfach und kostengünstig aus den entsprechenden 3-Halogen-alkylbenzolen, beispielsweise dem technisch verfügbaren 3-Fluortoluol, in zwei Syntheseschritten durch Nitrierung in 2,4-Stellung und nachfolgende Reduktion der Nitrogruppen gemäß dem nachstehenden Schema herstellen, wobei R und X die vorstehend angegebene Bedeutung haben: 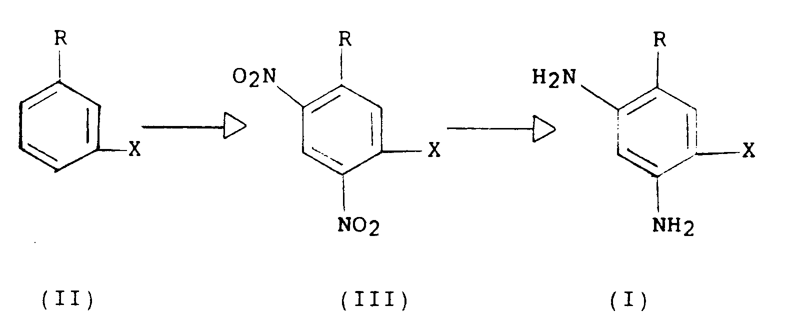
Für die Nitrierung kommen die in der organischen Synthese üblichen Nitrierungsreagenzien in Frage, wobei ein Gemisch aus Schwefelsäure und Salpetersäure bevorzugt ist. Für die Reduktion der Nitrogruppen kommen ebenfalls die für diesen Syntheseschritt bekannten Reagenzien, beispielsweise Wasserstoff in Gegenwart eines geeigneten Katalysators, in Betracht.

Gegenstand der vorliegenden Anmeldung ist weiterhin ein neues 5-Halogen-2,4-diamino-alkylbenzol der allgemeinen Formel 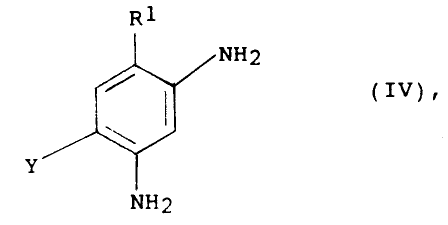
wobei R¹ einen geradkettigen oder verzweigten C₁- bis C₄-Alkylrest bedeuted und Y Fluor oder Brom darstellt, unter der Voraussetzung, daß Y nicht Brom bedeutet, wenn R¹ der Methylrest ist.

Als Beispiel für die neuen Verbindungen der Formel (IV) sei das 5-Fluor-2,4-diamino-toluol genannt.

Die nachfolgenden Beispiele sollen den Gegenstand der Erfindung näher erläutern.

### Beispiele

### Beispiel 1: Haarfärbelösung

0,80 g 5-Fluor-2,4-diamino-toluol
2,80 g 2-Methyl-1,4-diaminobenzolsulfat
0,60 g 4-(2'-Hydroxyethyl)amino-1,2-methylendioxybenzol
0,10 g m-Aminophenol
0,45 g 3-Amino-5-hydroxy-2,6-dimethoxypyridinhydrochlorid
0,40 g Natriumsulfit, wasserfrei
10,00 g Laurylalkohol-diglykolethersulfat (28 %ige wäßrige Lösung)
10,00 g Isopropanol
20,00 g Ammoniak (22 %ige wäßrige Lösung)
54,85 g Wasser, vollentsalzt
1̅0̅0̅,̅0̅0̅ g̅
50 g der vorstehenden Haarfärbelösung werden kurz vor der Anwendung mit 50 ml einer 6 %igen Wasserstoffperoxidlösung vermischt. Das Gemisch wird sodann auf blonde menschliche Haare aufgetragen. Nach einer Einwirkungszeit von dreißig Minuten bei vierzig Grad Celsius wird das Haar mit Wasser gespült und getrocknet. Das Haar ist schwarz gefärbt.

### Beispiel 2: Haarfärbemittel in Gelform

0,15 g 5-Fluor-2,4-diamino-toluol
1,10 g 2-(2'-Hydroxyethyl)-1,4-diaminobenzolsulfat
0,20 g Resorcin
0,05 g m-Aminophenol
0,40 g Natriumsulfit, wasserfrei
15,00 g Ölsäure
7,00 g Isopropanol
10,00 g Ammoniak (22 %ige wäßrige Lösung)
66,10 g Wasser
1̅0̅0̅,̅0̅0̅ g̅
Man vermischt kurz vor der Anwendung 50 g dieses Haarfärbemittels in Gelform mit 50 ml einer 6 %igen Wasserstoffperoxidlösung. Das Gemisch wird sodann auf blonde menschliche Haare aufgetragen. Nach einer Einwirkungszeit von dreißig Minuten bei 40 Grad Celsius wird das Haar mit Wasser gespült und sodann getrocknet. Das Haar hat eine natürliche mittelblonde Färbung angenommen.

### Beispiel A: Herstellung von 5-Fluor-2,4-diamino-toluol

### Stufe 1: 5-Fluor-2,4-dinitro-toluol

5,5 g 3-Fluortoluol werden mit einem erkalteten Gemisch von 10,9 ml konzentrierter H₂SO₄ und 9,3 ml konzentrierter HNO₃ nitriert. Die Mischung wird 24 Stunden lang bei Raumtemperatur gerührt und anschliessend auf Eis gegossen. Das ausgefallene Produkt wird abgesaugt mit H₂O gewaschen und nach Umkristallisation aus Alkohol werden 5,8 g (58 % der Theorie) schwach gelb gefärbte Kristalle mit einem Schmelzpunkt von 80 Grad Celsius erhalten.
¹H-NMR( D₆ -DMSO):
- δ =: 2,64 (s; 3 H, -CH₃) 7,85 (d; J = 12 Hz, 1 H, 6-H) 8,76 ppm (d; J = 7,1 Hz, 1 H, 3-H)
MS (70 eV): m/e = 200 (M⁺)
Stufe 2: 5-Fluor-2,4-diamino-toluol
2 g (0,01 ml) 5-Fluor-2,4-dinitro-toluol aus Stufe 1 werden mit katalytischen Mengen Palladium/Kohlenstoff (10 % Pd) in 50 ml absoluten Ethanol hydriert. Nach dem Abfiltrieren des Katalysators wird das Lösungsmittel vollständig abdestilliert und das erhaltene Produkt aus Toluol umkristallisiert. Man erhält 0,7 g (50 % der Theorie) 5-Fluor-2,4-diamino-toluol, das bei 112 Grad Celsius unter Zersetzung schmilzt.
¹H-NMR( D₆ -DMSO:

- δ =: 2,49 (s; 3 H, -CH₃) 4,36 (s; 2 H, NH₂) 4,54 (s; 2 H, NH₂) 6,03 (d; J = 8,6 Hz, 3-H) 6,54 ppm (d; J = 12 Hz, 6-H)
MS (70 eV): m/e = 140 (M⁺)
Alle in der vorliegenden Anmeldung angegebenen Prozentzahlen stellen, sofern nicht anders vermerkt, Gewichtsprozent dar.

## Patentansprüche

1. Mittel zur oxidativen Färbung von Haaren auf der Basis einer Entwicklersubstanz-Kupplersubstanz-Kombination sowie gegebenenfalls anderer Farbkomponenten und in Haarfärbemitteln üblichen Zusätzen, dadurch gekennzeichnet, daß es mindestens eine Kupplersubstanz der allgemeinen Formel 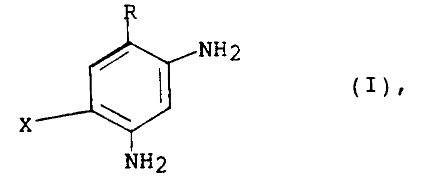 wobei R einen geradkettigen oder verzweigten C₁- bis C₄-Alkylrest bedeutet und X Fluor oder Brom darstellt, oder deren physiologisch verträgliches wasserlösliches Salz enthält.

2. Mittel nach Anspruch 1, dadurch gekennzeichnet, daß es die Kupplersubstanz der allgemeinen Formel (I) in einer Menge von 0,01 bis 5 Gewichtsprozent enthält.

3. Mittel nach Anspruch 1 oder 2, dadurch gekennzeichnet, daß es als Kupplersubstanz 5-Brom-2,4-diaminotoluol enthält.

4. Mittel nach Anspruch 1 oder 2, dadurch gekennzeichnet, daß es als Kupplersubstanz 5-Fluor-2,4-diaminotoluol enthält.

5. Mittel nach einem der Ansprüche 1 bis 4, dadurch gekennzeichnet, daß es zusätzlich mindestens eine weitere Kupplersubstanz enthält, welche ausgewählt ist aus Resorcin, 4-Chlorresorcin, 4,6-Dichlorresorcin, 2-Methylresorcin, 2-Amino-4-(2'-hydroxyethylamino)anisol, 2-Amino-4-ethylaminoanisol, 2,4-Diaminobenzylalkohol, m-Phenylendiamin, 5-Amino-2-methylphenol, 2,4-Diaminophenoxyethanol, 1-Naphthol, m-Aminophenol, 3-Amino-4-chlor-6-methylphenol, 3-Amino-2-methyl-phenol, 4-Amino-2-hydroxyphenoxyethanol, 4-Hydroxy-1,2-methylendioxybenzol, 4-(2'-Hydroxyethylamino)-1,2-methylendioxybenzol, 2,4-Diamino-5-ethoxytoluol, 4-Hydroxyindol, 3-Amino-5-hydroxy-2,6-dimethoxypyridin und 3,5-Diamino-2,6-dimethoxypyridin enthalten sein.

6. Mittel nach einem der Ansprüche 1 bis 5, dadurch gekennzeichnet, daß die Entwicklersubstanz ausgewählt ist aus 1,4-Diaminobenzol, 2,5-Diaminotoluol, 2,5-Diaminobenzylalkohol, 2-(2'-Hydroxyethyl)-1,4-diaminobenzol, 4-Aminophenol, 4-Amino-2-aminomethyl-phenol, 4-Amino-3-methylphenol, 4-Amino-2-methoxymethylphenol, 4-Amino-2-ethoxymethylphenol sowie Tetraaminopyrimidin oder deren physiologisch verträglichen Salzen.

7. Mittel nach einem der Ansprüche 1 bis 6, dadurch gekennzeichnet, daß die Gesamtmenge der Entwicklersubstanz-Kupplersubstanz-Kombination 0,1 bis 5,0 Gewichtsprozent beträgt.

8. Mittel nach einem der Ansprüche 1 bis 7, dadurch gekennzeichnet, daß es eine Farbkomponente enthält, welche ausgewählt ist aus 6-Amino-2-methylphenol, 2-Amino-5-methylphenol, Basic Violet 14 (C. I. 42 510), Basic Violet 2 (C. I. 42 520), 2-Nitro-1,4-diaminobenzol, 2-Amino-4-nitrophenol, 2-Amino-5-nitrophenol, 2-Amino-4,6-dinitrophenol, 2-Amino-5-(2'-hydroxyethylamino)-nitrobenzol, 4-(2'-Hydroxyethylamino)-3-nitrotoluol, 1-(2'-Ureidoethyl)-amino-4-nitrobenzol, Acid Brown 4 (C. I. 14 805), 1,4-Diaminoanthrachinon und 1,4,5,8-Tetraaminoanthrachinon.

9. 5-Halogen-2,4-diamino-alkylbenzol der allgemeinen Formel 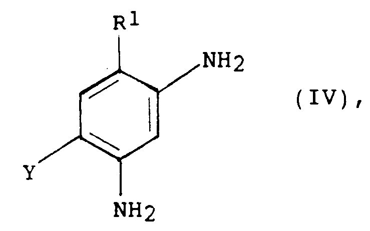 wobei R¹ einen geradkettigen oder verzweigten C₁-bis C₄-Alkylrest bedeutet und Y Fluor oder Brom darstellt, unter der Voraussetzung, daß Y nicht Brom bedeutet, wenn R¹ der Methylrest ist.

10. 5-Fluor-2,4-diamino-toluol

## Claims

1. Oxidative hair dyeing agent based on a developer substance-coupler substance combination as well as, optionally, other colouring components and additives conventional in hair colouring agents, characterised in that it contains at least one coupler substance of the general formula (I) 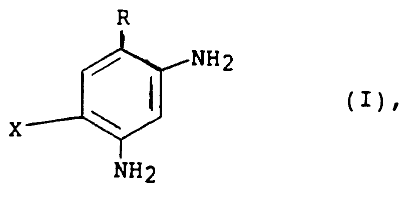 in which R is a straight chain or branched C₁-C₄ alkyl residue and X is fluorine or bromine, or the physiologically compatible water-soluble salt thereof.

2. Agent according to Claim 1, characterised in that it contains the coupler substance of the general formula (I) in an amount of between 0.01 and 5 weight %.

3. Agent according to Claim 1 or 2, characterised in that it contains 5-bromo-2,4-diaminotoluene as coupler substance.

4. Agent according to Claim 1 or 2, characterised in that it contains 5-fluoro-2,4-diaminotoluene as coupler substance.

5. Agent according to any one of Claims 1 to 4, characterised in that in addition it contains at least one further coupler substance which is selected from resorcinol, 4-chlororesorcinol, 4,6-dichlororesorcinol, 2-methylresorcinol, 2-amino-4-(2'-hydroxyethylamino)anisole, 2-amino-4-ethylamino-anisole, 2,4-diaminobenzyl alcohol, m-phenylenediamine, 5-amino-2-methylphenol, 2,4-diaminophenoxyethanol, 1-naphthol, m-aminophenol, 3-amino-4-chloro-6-methylphenol, 3-amino-2-methylphenol, 4-amino-2-hydroxyphenoxyethanol, 4-hydroxy-1,2-methylenedioxybenzene, 4-(2'-hydroxyethylamino)-1,2-methylenedioxybenzene, 2,4-diamino-5-ethoxytoluene, 4-hydroxyindole, 3-amino-5-hydroxy-2,6-dimethoxypyridine and 3,5-diamino-2,6-dimethoxypyridine.

6. Agent according to any one of Claims 1 to 5, characterised in that the developer substance is selected from 1,4-diaminobenzene, 2,5-diaminotoluene, 2,5-diaminobenzylalcohol, 2-(2'-hydroxyethyl)-1,4-diaminobenzene, 4-aminophenol, 4-amino-2-aminomethyl-phenol, 4-amino-3-methylphenol, 4-amino-2-methoxymethylphenol, 4-amino-2-ethoxymethylphenol as well as tetraaminopyrimidine or the physiologically compatible salts thereof.

7. Agent according to any one of Claims 1 to 6, characterised in that the total amount of developer substance - coupler substance combination is 0.1 to 5.0 weight %.

8. Agent according to any one of Claims 1 to 7, characterised in that it contains a colouring component which is selected from 6-amino-2-methylphenol, 2-amino-5-methylphenol, Basic Violet 14 (C.I. 42 510), Basic Violet 2 (C.I. 42 520), 2-nitro-1,4-diaminobenzene, 2-amino-4-nitrophenol, 2-amino-5-nitrophenol, 2-amino-4,6-dinitrophenol, 2-amino-5-(2'-hydroxyethylamino)-nitrobenzene, 4-(2'-hydroxyethylamino)-3-nitrotoluene, 1-(2'-ureidoethyl)-amino-4-nitrobenzene, Acid Brown 4 (C.I. 14 805), 1,4-diaminoanthraquinone and 1,4,5,8-tetraaminoanthraquinone.

9. 5-halogen-2,4-diamino-alkylbenzene of the general formula (IV) 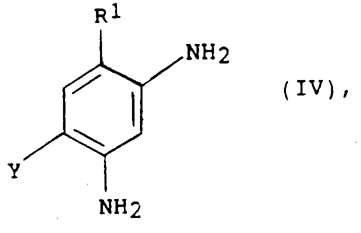 in which R¹ is a straight chain or branched C₁ - C₄ alkyl residue and Y is fluorine or bromine provided that Y is not bromine if R¹ is the methyl residue.

10. 5-fluoro-2,4-diamino-toluene.

## Revendications

1. Agent de coloration oxydant des cheveux à base d'une combinaison copulant-développeur ainsi que le cas échéant d'autres composants de coloration, et des additifs usuels dans les agents de coloration des cheveux caractérisé en ce qu'il contient au moins une substance de copulation de formule générale 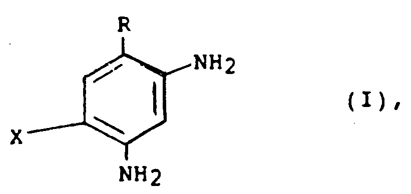 dans laquelle R est un reste alkyle à chaîne droite ou ramifiée en C₁-C₄, et X représente le fluor ou le brome ou son sel soluble dans l'eau compatible physiologiquement.

2. Agent selon la revendication 1, caractérisé en ce qu'il contient le copulant de formule générale (I) à raison de 0,01 à 5 % en poids.

3. Agent selon la revendication 1 ou 2, caractérisé en ce qu'il contient comme copulant du 5-bromo-2, 4-diaminotoluène.

4. Agent selon la revendication 1 ou 2, caractérisé en ce qu'il contient comme copulant du 5-fluoro-2, 4-diaminotoluène.

5. Agent selon l'une des revendications 1 à 4, caractérisé en ce qu'il contient en outre au moins un autre copulant qui est choisi parmi la résorcine, la 4-chloro-résorcine, la 4,6-dichlororésorcine, la 2-méthyl-résorcine, le 2-amino-4-(2'-hydroxyéthylamino) anisol, le 2-amino-4-éthylaminoanisol le 2,4-diaminobenzylalcool, le m-phenylènediamine, le 5-amino-2-methylphénol le 2,4-diaminophénoxyéthanol, le 1-naphtol, le m-aminophénol, le 3-amino-4-chloro-6-méthylphénol le 3-amino-2-méthylphènol, le 4-amino-2-hydroxy-phénoxyéthanol, le 4-hydroxy-1,2-méthylènedioxybenzène, le 4-(2'-hydroxyéthylamino)-1,2-méthylène-dioxybenzène, le 2,4-diamino-5-éthoxytoluène, le 4-hydroxyindol, le 3-amino-5-hydroxy-2,6-diméthoxypyridine, et la 3,5-diamino-2,6-diméthoxypyridine.

6. Agent sel on l'une des revendications 1 à 5, caractérisé en ce que le développeur est choisi parmi le 1,4-diaminobenzène, le 2,5-diaminotoluène, le 2,5-diaminobenzylalcool, le 2-(2'hydroxy-éthyl)-1,4-diaminobenzène, le 4-aminophénol, le 4-amino-2-aminométhyl-phénol, le 4-amino-3-méthyl-phénol, le 4-amino-2-méthoxyméthylphénol, le 4-amino-2-éthoxyméthylphénol, ainsi que la tétra-aminopyrimidine ou leurs sels physiologiquement compatibles.

7. Agent selon l'une des revendications 1 à 6, caractérisé en ce que la quantité totale de la combinaison copulant-développeur va de 0,1 à 5,0 % en poids.

8. Agent sel on l'une des revendications 1 à 7, caractérisé en ce qu'il contient un composant de coloration qui est choisi parmi le 6-amino-2-méthylphénol, le 2-amino-5-méthylphénol, le Basic Violet 14 (C.I.42 510), le Basic Violet 2 (C.I. 42 520), le 2-nitro-1,4-diaminobenzène, le 2-amino-4-nitrophénol, le 2-amino-5-nitrophénol, le 2-amino-4,6-dinitrophénol, le 2-amino-5-(2'-hydroxy-éthylamino)nitrobenzène, le-4-(2'-hydroxyéthyl-amino)-3-nitrotoluène, le 1-(2'-uréidoéthyl)-amino-4-nitrobenzène, l'Acide Brown 4 (C.I. 14805), la 1,4-diaminoanthraquinone, et la 1,4,5,8-tétraaminoanthraquinone.

9. Le 5-halogéno-2,4-diamino-alkylbenzène de formule générale : 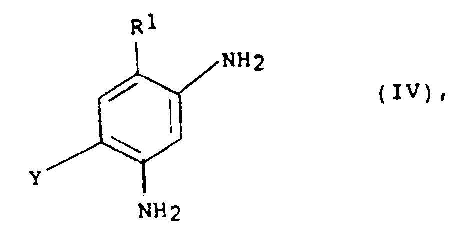 dans laquelle R1 représente un reste alkyle à chaîne droite ou ramifiée en C₁ à C₄, et Y le fluor ou le brome, sous la condition que Y n'est pas le brome quand R¹ est un reste méthyle.

10. Le 5-fluoro-2,4-diamino-toluène.
